Europäisches Patentamt

European Patent Office

Office européen des brevets

(19)

(11) Veröffentlichungsnummer: **0 065 804**
**B1**

(12) # EUROPÄISCHE PATENTSCHRIFT

(45) Veröffentlichungstag der Patentschrift:
**12.12.84**

(21) Anmeldenummer: **82200607.8**

(22) Anmeldetag: **17.05.82**

(51) Int. Cl.$^3$: **C 07 C 103/365**, C 07 C 102/00

(54) Verfahren zur Herstellung von N-Benzyl-N-isopropylpivaloylamid.

(30) Priorität: **22.05.81 DE 3120361**

(43) Veröffentlichungstag der Anmeldung:
**01.12.82 Patentblatt 82/48**

(45) Bekanntmachung des Hinweises auf die Patenterteilung:
**12.12.84 Patentblatt 84/50**

(84) Benannte Vertragsstaaten:
**AT CH DE FR GB IT LI NL**

(56) Entgegenhaltungen:
**FR - A - 1 211 287**
**FR - A - 2 259 812**
**US - A - 3 647 876**

(73) Patentinhaber: **CHEMISCHE WERKE HÜLS AG,
Postfach 1320, D-4370 Marl 1 (DE)**

(72) Erfinder: **Bellut, Hans, Dr., Bergstrasse 50,
D-4408 Dülmen (DE)**

(74) Vertreter: **Steil, Hanna, Dipl.-Chem., RSP PATENTE -
PB 15 Postfach 1320, D-4370 Marl 1 (DE)**

BUNDESDRUCKEREI BERLIN

**0 065 804**

**Beschreibung**

Die Erfindung betrifft ein neues Verfahren zur Herstellung von N-Benzyl-N-isopropylpivaloylamid aus Benzylhalogenid, Isopropylamin und Pivaloylchlorid.

Es sind verschiedene Verfahren zur Herstellung von N-Benzyl-N-isopropylaminen aus Benzylhalogeniden und Alkylaminen bekannt.

Diese Verfahren weisen jedoch eine Reihe von Nachteilen auf. So ist z. B. vielfach ein organisches Lösungsmittel erforderlich. Üblicherweise muß man entweder mit einem hohen Überschuß an primärem Amin arbeiten oder eine Schutzgruppe einführen, um die Bildung größerer Mengen tertiärer Amine zu vermeiden.

Da das eingesetzte Alkylamin nicht nur als Nukleophil, sondern auch als HCl-Akzeptor fungiert, wird es laufend der weiteren Reaktion entzogen. Die Gegenwart des entstandenen Hydrochlorids wirkt sich seinerseits ungünstig auf den weiteren Reaktionsablauf aus. Es ist vorgeschlagen worden, der Reaktionsmischung Triethylamin oder Pyridin zum Abfangen der Halogenwasserstoffsäure zuzusetzen. Damit aber ergeben sich neue Probleme der Abtrennung und Regenerierung der organischen Hilfsbasen.

Es ist nach der deutschen Patentschrift DE-PS 2 104 857 bekannt, N-Benzyl-N-isopropylpivaloylamid dadurch herzustellen, daß man Pivaloylchlorid zu einer Lösung von N-Benzyl-N-isopropylamin und Triethylamin in Benzol tropft.

Auch bei dieser Reaktion ist der Einsatz der organischen Hilfsbase umständlich, da sie nach der Reaktion regeneriert werden muß. Die Reaktion erfordert eine Reaktionszeit von wenigstens 18 Stunden.

Die Ausbeute kann nicht als befriedigend angesehen werden.

Die vorliegende Erfindung bezweckt nun eine Erhöhung der Selektivität und Ausbeute an N-Benzyl-N-isopropylpivaloylamid bei gleichzeitig vereinfachter Verfahrensweise.

Der Erfindung liegt die Aufgabe zugrunde, die bekannten Verfahren so zu modifizieren, daß in wäßrigen Systemen gearbeitet und dennoch die Hydrolyse des Benzylhalogenids sowie des Pivaloylchlorids vermieden werden kann. Es wurde übrraschenderweise ein Verfahren gefunden, mit dem die aufgeführten Schwierigkeiten und Nachteile vermieden werden können.

Gegenstand der Erfindung ist ein Verfahren zur Herstellung von N-Benzyl-N-isopropylpivaloylamid aus Benzylhalogenid, Isopropylamin und Pivaloylchlorid, das dadurch gekennzeichnet ist, daß man 1 Mol Benzylhalogenid mit 1,5 bis 3, vorzugsweise mit 2 Mol Isopropylamin und 1,25 bis 1,75, vorzugsweise 1,5 Mol Alkalilauge in Form einer wäßrigen Lösung umsetzt, deren Konzentration so gewählt wird, daß das bei der Reaktion gebildete Alkalihalogenid gerade noch gelöst bleibt, die Reaktionsmischung für 2—3 Stunden bei einer Temperatur von 30° bis 50°C hält, das durch Destillation der organischen Phase erhaltene N-Benzyl-N-isopropylamin in einem Zweiphasensystem, bestehend aus

a) Toluol und/oder anderen alkylierten Aromaten mit bis zu insgesamt 10 C-Atomen und
b) 20%iger wäßriger Natronlauge, wobei die Menge so bemessen sein sollte, daß nicht nur die bei der Reaktion gebildete Salzsäure quantitativ abgefangen wird, sondern daß auch die wäßrige Phase nach Beendigung der Reaktion alkalisch bleibt,

bei einer Temperatur von 0° bis 60°C vorlegt, Pivaloylchlorid zusetzt, für eine Stunde bei einer Temperatur zwischen 0 und 60°C hält und danach das entstandene Produkt auf übliche Weise isoliert.

Als Benzylhalogenid kann das Chlorid, Bromid und Jodid eingesetzt werden. Pro Mol Benzylhalogenid werden 1,5 bis 3, vorzugsweise 2 Mol Isopropylamin eingesetzt. Bei weiterer Erhöhung des Überschusses an Isopropylamin wird der Anteil des in der organischen Phase gelösten Wassers größer und damit die Aufarbeitung erschwert.

Die Menge des eingesetzten Alkalihydroxids sollte über der stöchiometrisch berechneten liegen, um sowohl einen guten Neutralisationseffekt zu erzielen als auch die Löslichkeit der Amine in der wäßrigen Phase während der Reaktion möglichst gering zu halten. Die optimale Ausgangskonzentration der Alkalilösung läßt sich berechnen, indem man die Wassermenge zum Lösen der Lauge so wählt, daß das bei der Reaktion gebildete Halogenid neben dem unumgesetzten Alkalihydroxid gerade noch gelöst wird. Gute Erfahrungen wurden mit der 1,25- bis 1,8fachen Menge Alkalihydroxid in 15%iger wäßriger Lösung gemacht.

Die Reaktion wird im Temperaturbereich zwischen 20° und 50°C, zweckmäßigerweise bei 40°C durchgeführt. Insbesondere bei größeren Ansätzen muß zu Beginn der Reaktion leicht gekühlt, gegen Ende der Reaktion geheizt werden. Die Reaktion wird vorteilhafterweise ohne weitere Lösungsmittel durchgeführt. Die zeitliche Verfolgung der Reaktion durch Bestimmung des Halogenidgehaltes der wäßrigen Phase ermöglicht es, den Prozeß bei einem bestimmten Umsetzungsgrad oder bei Überschreitung eines bestimmten Verhältnisses von sekundärem zu tertiärem Amin abzubrechen. Im allgemeinen ist dies nach etwa 2 bis 3 Stunden der Fall.

Das gebildete N-Benzyl-N-isopropylamin läßt sich auf einfache Weise durch Vakuumdestillation der nach Phasentrennung erhaltenen organischen Phase isolieren. Daneben ist es möglich, das im Überschuß eingesetzte Isopropylamin quantitativ zurückzugewinnen.

2

**0 065 804**

Zur Erzielung einer hohen Ausbeute empfiehlt es sich, das erhaltene sekundäre Amin mit 0,5 bis 0,8, vorzugsweise mit 0,67 Äquivalenten Pivaloylchlorid umzusetzen und die 20%ige wäßrige Natronlauge in einem 1,2fachen Überschuß an Natriumhydroxid, bezogen auf das Säurechlorid anzuwenden. Auf diese Weise lassen sich Ausbeuten von 99%, bezogen auf eingesetztes N-Benzyl-N-isopropylamin erzielen. Man kann indessen auch mit einem 1,2- bis 2fachen, zweckmäßigerweise mit einem 1,5fachen Überschuß an N-Benzyl-N-isopropylamin arbeiten und die 20%ige wäßrige Natronlauge in einem 1,2fachen Überschuß an Natriumhydroxid, bezogen auf das Säurechlorid, anwenden. Diese Variante hat den Vorteil, daß das im Überschuß eingesetzte N-Benzyl-N-isopropylamin qantitativ zurückgewonnen werden kann.

Die Reaktion des durch Destillation der organischen Phase erhaltenen N-Benzyl-N-isopropylamins mit Pivaloylchlorid wird üblicherweise so ausgeführt:

Zunächst wird das sekundäre Amin in einem Zweiphasensystem, bestehend aus

a) Toluol und/oder anderen alkylierten Aromaten mit bis zu insgesamt 10 C-Atomen und
b) 20% wäßriger Natronlauge

bei einer Temperatur von 0 bis 60° C vorgelegt. Die Alkalimenge sollte so bemessen sein, daß nicht nur die bei der Reaktion gebildete Salzsäure quantitativ abgefangen wird, sondern daß auch die wäßrige Phase nach Beendigung der Reaktion alkalisch bleibt. Anschließend setzt man Pivaloylchlorid zu und läßt eine Stunde ausreagieren. Die Reaktionstemperatur liegt zwischen 0 und 60° C, vorzugsweise bei 10° C.

Die Abtrennung des N-Benzyl-N-isopropylpivaloylamids kann nach bekannten Methoden erfolgen, beispielsweise durch Destillation.

Nach dem vorliegenden Verfahren wird im Vergleich zum Stand der Technik eine wesentlich bessere Ausbeute erzielt.

Die Reaktionszeiten sind deutlich verkürzt. Gleichzeitig gestatten die erfindungsgemäß eingesetzten Zweiphasensysteme nicht nur eine erheblich vereinfachte Isolierung der Reaktionsprodukte, sondern auch die nahezu quantitative Rückgewinnung nicht umgesetzter Einsatzstoffe. Die nach Neutralisierung erhaltenen wäßrigen Phasen können bedenkenlos dem Abwasser zugeführt werden. N-Benzyl-N-isopropylpivaloylamid findet bekanntermaßen als Herbizid weite Verwendung.

Die nachfolgenden Beispiele dienen zur Veranschaulichung des erfindungsgemäßen Verfahrens:

Beispiel 1

Zu einer gerührten Mischung aus 236 g (4 Mol) Isopropylamin und einer Lösung von 120 g (3 Mol) Natriumhydroxid in 680 ml Wasser wurden unter leichter Kühlung 252 g (2 Mol) Benzylchlorid zugetropft und anschließend unter Erwärmung 3 Stunden miteinander verrührt, so daß die Temperatur konstant 40° C betrug. Anschließend wurde die organische Phase über Kühlfallen fraktionierend destilliert. Man erhielt 43 g (0,73 Mol) Isopropylamin ($n_D^{20} = 1,3840$), 60 g eines Gemisches aus Isopropylamin und Wasser, das der wäßrigen Phase zugeschlagen wurde, und schließlich ein Azeotrop aus Wasser und N-Benzyl-N-isopropylamin. Letzteres wurde erneut dem Sumpf der organischen Phase zugeführt. 2,5 g N-Benzyl-N-isopropylaminhydrochlorid wurden abgetrennt. Die anschließende Destillation im Vakuum ergab folgende 4 Fraktionen:

1. Sdp.$_1$ = 40° C    $n_D^{21} = 1,5035$    26 g (leicht verunreinigtes N-Benzyl-N-isopropylamin)
2. Sdp.$_1$ = 40—44° C    $n_D^{21} = 1,5008$    209 g (N-Benzyl-N-isopropylamin)
3. Sdp.$_1$ = 44—100° C    $n_D^{21} = 1,5175$    2 g (Zwischenfraktion)
4. Sdp.$_1$ = 110° C    $n_D^{21} = 1,5448$    30 g (N,N-Dibenzyl-N-isopropylamin)

Aus der wäßrigen Phase ließen sich durch Wasserdampfdestillation 78 g Isopropylamin austreiben. Insgesamt konnten also 121 g (2,0 Mol) Isopropylamin zurückgewonnen werden. Neben 30 g tertiären Amin sind 233 g (1,56 Mol) N-Benzyl-N-isopropylamin entstanden.

Die Ausbeute betrug 78%.

Eine Lösung von 50 g (1,25 Mol) Natriumhydroxid in 290 ml Wasser wird bei 10° C vorgelegt und mit einer Lösung von 233 g (1,56 Mol) N-Benzyl-N-isopropylamin in 260 ml Toluol gerührt. Zu diesem Zweiphasensystem läßt man innerhalb einer Stunde eine Lösung von 126 g (1,04 Mol) Pivalinsäurechlorid in 130 ml Toluol zutropfen. Nach einer weiteren Stunde trennt man die beiden flüssigen Phasen. Aus der organischen Phase wurden zunächst Reste von Wasser, das Lösungsmittel Toluol sowie 88 g (0,59 Mol) N-Benzyl-N-isopropylamin im Vakuum abdestilliert. Das zurückbleibende N-Benzyl-N-isopropylpivaloylamid wurde durch einmalige Destillation (Sdp.$_1$ = 130° C) in einer Reinheit von 99,6% (n  = 1,5120) erhalten.

Die Ausbeute betrug 198 g (0,86 Mol) oder 82%.

3

## Beispiel 2

Analog zu Beispiel 1 wurden 233 g (1,56 Mol) N-Benzyl-N-isopropylamin erhalten. Sodann wurde eine Lösung von 50 g (1,25 Mol) Natriumhydroxid in 290 ml Wasser bei 10°C vorgelegt und mit einer Lösung von 233 g (1,56 Mol) N-Benzyl-N-isopropylamin in 260 ml Toluol gerührt. Zu diesem Zweiphasensystem läßt man innerhalb einer Stunde eine Lösung von 227 g (1,88 Mol) Pivalinsäurechlorid in 230 ml Toluol zutropfen. Man verfährt nun, wie in Beispiel 1 beschrieben, und erhält 354 g (1,54) Mol N-Benzyl-N-isopropylpivaloylamid, was einer Ausbeute von 99% entspricht.

## Beispiel 3

Analog zu Beispiel 1 wurden in halbtechnischem Maßstab 3200 g 15%ige (12 Mol) Natronlauge, 944 g (16 Mol) Isopropylamin und 1008 g (8,6 Mol) Benzylchlorid umgesetzt. Die Ausbeute an N-Benzyl-N-isopropylamin betrug 83,3 %. Eine abschließende Destillation des N-Benzyl-N-isopropylpivaloylamids war nicht erforderlich. Das Produkt fiel bei einer Reinheit von 99,8% in 87%iger Ausbeute an.

**Patentansprüche**

1. Verfahren zur Herstellung von N-Benzyl-N-isopropylpivaloylamid aus Benzylhalogenid, Isopropylamin und Pivaloylchlorid, dadurch gekennzeichnet, daß man
1 Mol Benzylhalogenid mit 1,5 bis 3, vorzugsweise mit 2 Mol Isopropylamin und 1,25 bis 1,75, vorzugsweise mit 1,5 Mol Alkalilauge in Form einer wäßrigen Lösung umsetzt, deren Konzentration gerade so gewählt wird, daß das bei der Reaktion gebildete Alkalihalogenid gerade noch gelöst bleibt,
die Reaktionsmischung für 2 bis 3 Stunden bei einer Temperatur von 30 bis 50°C hält,
das durch Destillation der organischen Phase erhaltene N-Benzyl-N-isopropylamin in einem Zweiphasensystem, bestehend aus

a) Toluol und/oder anderen alkylierten Aromaten mit bis zu insgesamt 10 C-Atomen und
b) 20%iger wäßriger Natronlauge, wobei die Menge so bemessen sein sollte, daß nicht nur die bei der Reaktion gebildete Salzsäure quantitativ abgefangen wird, sondern daß auch die wäßrige Phase nach Beendigung der Reaktion alkalisch bleibt,

bei einer Temperatur von 0 bis 60°C vorlegt, Pivaloylchlorid zusetzt, für eine Stunde bei einer Temperatur zwischen 0 und 60°C hält und danach das entstandene Produkt auf übliche Weise isoliert.
2. Verfahren nach Anspruch 1, dadurch gekennzeichnet, daß man das gebildete N-Benzyl-N-isopropylamin mit 0,5 bis 0,8, vorzugsweise mit 0,67 Äquivalenten Pivaloylchlorid umsetzt und die 20%ige wäßrige Natronlauge einen 1,2fachen Überschuß an Natriumhydroxid, bezogen auf das Säurechlorid, enthält.
3. Verfahren nach Anspruch 1, dadurch gekennzeichnet, daß man das gebildete N-Benzyl-N-isopropylamin mit 1,2 bis 2, vorzugsweise mit 1,5 Äquivalenten Pivaloylchlorid versetzt und die 20%ige wäßrige Natronlauge einen 1,2fachen Überschuß an Natriumhydroxid, bezogen auf das Säurechlorid, enthält.

**Claims**

1. A process for the production of N-benzyl-N-isopropylpivaloylamide from a benzyl halide, isopropylamine and pivaloyl chloride, characterised in that 1 mole of benzyl halide is reacted with 1.5 to 3, preferably 2, moles of isopropylamine and 1.25 to 1.75, preferably 1.5, moles of alkali metal hydroxide in the form of an aqueous solution,the concentration of which is so chosen that the alkali metal halide formed during the reaction only just remains in solution, the reaction mixture is kept for 2 to 3 hours at a temperature of 30 to 50°C, the N-benzyl-N-isopropylamine obtained by distillation of the organic phase is introduced into a two-phase system consisting of

a) toluene and/or any other alkylated aromatic having up to 10 C atoms in total and
b) 20% strength aqueous sodium hydroxide solution, the amount being so chosen that not only is the hydrochloric acid formed during the reaction neutralised quantitatively, but the aqueous phase also remains alkaline after completion of the reaction,

at a temperature of 0 to 60°C, pivaloyl chloride is added, the mixture is kept for one hour at a temperature of between 0 and 60°C and thereafter the product formed is isolated in a conventional manner.
2. A process according to claim 1, characterised in that the N-benzyl-N-isopropylamine formed is reacted with 0.5 to 0.8, preferably 0.67, equivalent of pivaloyl chloride and the 20% strength aqueous sodium hydroxide solution contains a 1.1-fold excess of sodium hydroxide over the acid chloride.

3. A process according to claim 1, characterised in 1.2—2, preferably 1.5, equivalents of pivaloyl chloride are added to the N-benzyl-N-isopropylamine formed and that the 20% strength aqueous sodium hydroxide solution contains a 1.2-fold excess of sodium hydroxide over the acid chloride.

## Revendications

1. Procédé de préparation de N-benzyl-N-isopropyl-pivaloylamide en partant d'halogénure de benzyle, d'isopropylamine et de chlorure de pivaloyle, caractérisé par le fait que l'on fait réagir une mole d'halogénure de benzyle sur 1,5 à 3, de préférence 2 moles, d'isopropylamine et sur 1,25 à 1,75, de préférence 1,5 mole, de lessive alcaline sous la forme d'une solution aqueuse dont on choisit la concentration exactement de façon telle que l'halogénure alcalin formé dans la réaction reste encore tout juste dissous, que l'on maintient le mélange réactionnel pendant 2 à 3 heures à une température de 30 à 50°C, que l'on place la N-benzyl-N-isopropyllamine obtenue par distillation de la phase organique, à une température de zéro à 60°C, dans un système à deux phases formé:

a) de toluène et/ou d'autres composés aromatiques lakylés contenant au maximum un total de 10 atomes de carbone et
b) de lessive de soude aqueuse à 20%, la quantité devant être calculée de façon telle que non seulement l'acide chlorhydrique formé lors de la réaction soit fixé quantitativement, mais que la phase aqueuse reste également alcaline après la fin de la réaction,

que l'on ajoute du chlorure de pivaloyle, que l'on maintient pendant une heure à une température comprise entre zéro et 60°C et qu'on isole ensuite de façon usuelle le produit formé.

2. Procédé selon la revendication 1, caractérisé par le fait que l'on fait réagir la N-benzyl-N-isopropylamine sur 0,5 à 0,8, de préférence sur 0,67 équivalent de chlorure de pivaloyle et que la lessive de soude aqueuse à 20% contient un excès d'hydroxyde de sodium de 1,2 fois, relativement au chlorure d'acide.

3. Procédé selon la revendication 1, caractérisé par le fait que l'on ajoute à la N-benzyl-N-isopropylamine formée de 1,2 à 2, de préférence de 1,5 équivalent de chlorure de pivaloyle et que la lessive de soude aqueuse à 20% contient un excès d'hydroxyde de sodium de 1,2 fois relativement au chlorure d'acide.